# EUROPEAN PATENT SPECIFICATION

(11) **EP 0 871 366 B1**
(45) Date of publication and mention of the grant of the patent: **08.06.2005**
(21) Application number: 96912338.9
(22) Date of filing: 09.05.1996
(51) Int. Cl.: A23C 9/00, A23L 1/31, G01N 33/04, G01N 33/68, G01N 33/487, A01K 67/02, C12Q 1/68, A61K 35/20, C07K 16/18

(54) **FOOD PRODUCT AND PROCESS**
NAHRUNGSMITTEL UND HERSTELLUNGSVERFAHREN
PRODUIT ALIMENTAIRE ET PROCEDE

(30) Priority: 16.05.1995 NZ 27213395
(43) Date of publication of application: 21.10.1998
(73) Proprietor: A2 Corporation Limited, Dunedin (NZ)
(72) Inventor: McLachlan, Corran Norman Stuart, Devonport North Shore 1309 (NZ)
(74) Representative: Lyons, Andrew John
(86) International application number: PCT/NZ1996/000039
(87) International publication number: WO 1996/036239

(56) References cited:
- EP-A- 0 631 731
- WO-A-96/14577
- AU-A- 7 659 094
- DATABASE BIOSIS [Online] BIOSCIENCES INFORMATION SERVICE, PHILADELPHIA, PA, US; 1993 BUMBERGER ELISABETH ET AL: "Bitter taste of enzymic hydrolysates of casein: I. Isolation, structural and sensorial analysis of peptides from tryptic hydrolysates of beta-casein." Database accession no. PREV199497029525 XP002162213 & ZEITSCHRIFT FUER LEBENSMITTEL-UNTERSUCHUNG UND -FORSCHUNG, vol. 197, no. 1, 1993, pages 14-19, ISSN: 0044-3026

## Description

### FIELD OF THE INVENTION

This invention relates to the removal, or the production of immunoglobulins against, immunosuppressant substances present, or, produced from the milk of animals of the genus Bos, and more particularly the domestic dairy species of the group Bos taurus and their crosses with the group Bos Indicus, which are used for milk production, and which contain specific casein alleles.

### BACKGROUND OF THE INVENTION

### Description of the Background Art

It has long been understood that the early lactation mammary secretions of certain species, known as colostrum, contains substances that prevent disease, whilst the immune system of the young of the species is developing. This is particularly true of the ruminants, such as the Bos family. However the ingestion of colostrum is not essential in the human. These substances were identified as proteins (globulins) with immuno-properties which became known as immunoglobulins, (B L Larsen Immunoglobulins of Mammary Secretions in Advanced Dairy Chemistry Volume 1 Proteins. Ed. PF Fox Elsevier,1992).

Immunoglobulins are present in the serum and mammary secretions of all mammalian species as part of the immune defence system of the animal. The immunoglobulins are also known as antibodies and are produced by the body's immune system in response to the presence of substances called antigens, including a wide range of molecules, bacteria, viruses, cells and particles that do not express specific markers of 'self' called histocompatibility antigens.

Molecular antigens are largely peptides, proteins and carbohydrates. The classic immune response involves the production of antibodies capable of neutralising these antigens.

The term antigen is now widely used to indicate any molecule that can be specifically recognised by the adaptive elements of the immune system, that is by both B cells, which produce immunoglobulins and T cells which release substances such as cytokines, (Immunology, 3rd Edition ,Ed. I Roitt, J Brostoff, D Male, Mosby, London, 1993).

There are five classes, or isotypes, of immunoglobulins all of which have a similar basic structure, but have differences in their organisational structure as well as the amino acid sequences present and carbohydrate groups present. In addition to the immunoglobulins there are present related immune system proteins. These are known as complement and they are a complex group of proteins which assist the function of antibodies. Their properties are described in the above texts. There are at least 11 proteins in the complement group some of which are expected to be present in milk at the milligram per 100 millilitre level.

There are numerous patents that have been filed which seek to:
1. isolate the immunoglobulins present in mammary secretions, particularly colostrum but also including milk and products derived from milk such as whey. Generally the species involved is the domestic cow, Bos taurus, but it may include sheep or goats.
2. produce an "immune milk" or "health food" incorporating the immunoglobulin proteins, either as a result of stimulating the milk producer's immune system by the addition or injection of substances into the animal's body, either once or systematically, which result in an immune response, or by concentrating the small amounts of immunoglobulins that are naturally present in milk -derived products. In the former case the immunoglobulins may be specific responses to the injection of pathogenic bacteria into the milk-producing animals.

### Examples of these patents include:

Japanese patent (1988) JP 63-133941, Hori T, Nishimoto K, Kimura M, Yommazaki N, describes a process in which immunoglobulins are collected by ultrafiltration from whey, the by-product of cheese or casein manufacture. The immunoglobulin content of powder derived from this process was found to contain about ten times the immunoglobulin content of dried human milk.

UK Patent Application (1987) GB 2 179 947 Monsan PFE, Thibault PA, Brossad C. Bruvier CSJ describes a process for the extraction of proteins, preferably lactoferrin or immunoglobulins from whey comprising concentration of the whey using ultrafiltration with a polysulphone membrane (with MW cut-off 25,000-50,000) followed by diafiltration. The retentate is then subject to adsorption of the retained proteins by ion exchange treatment using a weak cationic carboxymethyl resin at pH 5-8.5 and preferably at 7-8; and elution at the same pH.

European Patent Application (1984) EP 0 102 831 A¹ , Linggood MA, Porter P, Powell JR describes the immunisation of host animals with a range of E. Coli implicated in human gastroenteric disease and the production of immunoglobulins, and a synthetic milk containing the immunoglobulins that are specific responses to the inoculation of the host.

UK Patent Application (1987) 8729031, to R C Bottomley claims the production of a whey protein concentrate rich in immunoglobulins by the use of ultra-filtration through a membrane having a cut-off of 500,000 daltons which retains the immunoglobulins, or by subjecting whey to the action of an anion exchange resin which does not remove immunoglobulins so causing an increase in their concentration in the effluent.

European patent application (1989) EP 0 336 694 Beck LR, describes a process for extracting an anti-inflammatory factor from cow or ewe milk, taken from animals that have been previously immunised by the administration of bacterial antigens. The anti-inflammatory factor is then extracted from whey that has been subjected to ion-exchange chromatography and molecular sieve chromatography.

US patent (1992) 5 106 618 Beck LR, Kotler DP describes the production of a 'hyperimmune' milk obtained by inoculating a milk-producing animal with a non-protozoan bacterial antigens, collecting the milk from the animal and the pasteurising and concentrating prior to use.

US patents (1989) 4 879 110 and (1993) 5 194 255 Beck LR, Stolle RJ, describe a method for inducing the production of a milk anti-hypertensive factor in an animal such as a cow by injecting bacterial antigens into the animal. The anti-hypertension factor is isolated by (1) removing from the milk molecules having a molecular weight greater than 10,000 daltons;(2) fractionating by ion-exchange chromatography the effluent to obtain a negatively charged fraction;(3) fractionating the negatively charged material eluted from the ion-exchange column using molecular sieve chromatography and isolating the hypertensive fraction from the latter step by isoelectric precipitation.

US patent (1980) 4,216,236 Mueller HR, Legier CN, Secretin MC, Blonay CN claims the incorporation of soluble proteins obtained from whey using an ultrafiltration step with membranes having a molecular weight cut-off between 1000 and 500,000 incorporating immunoglobulins or to which immunoglobulin powder or concentrate has been added.

US patent (1984) 4 490 290 Ganni MM, May K, Porter P, describes the recovery of one or more milk immunoglobulins by passing the milk through a re-usable immunoadsorbent column comprising an insoluble carrier material to which is bound a low-affinity monoclonal antibody specific to the antibody(ies) but not specific to any other common constituent of milk. The bound immunoglobulin(s) are released by eluting the immunosorbent with 4 M MgCl₂.

### Problem

Notwithstanding all these patents and the claimed benefits of their products there is a considerable body of evidence that links milk particularly of the Bos taurus, the domestic cow with allergy problems with young children, asthma, chronic immune disorders such as diabetes mellitis, and atherosclerosis. Recent studies have also linked increased consumption of casein with the formation of hepatic tumours in rats, due it appears to a depressed NK cell cytotoxic activity, Bell RC, et al Nutr Cancr 22:151-162,(1994).

To date it has not been possible to identify any particular fraction or molecule that is responsible for disorders such as atherosclerosis, although the consumption of animal fats and their associated saturated fatty acids have been claimed to either cause, or contribute to, coronary heart disease, hypertension and obesity as is set out in most medical texts on these subjects and the Surgeon-General's Report on Nutrition and Health, DHHS Publication No 88-50210 (1988).

### OBJECT

It is an object of this invention to provide an improved food product and/or process or one which will at least provide the public with a useful choice.

### DEFINITIONS

"β-casein A¹ Allele" is a term used herein in reference to one of the variant forms of the β-casein gene. Expression of the A¹ allele results in the production of "β-casein A¹".

Where reference is made to the presence of the β-casein A¹ allele in an individual or population it encompasses both homozygous and heterozygous genotypes with respect to that allele.

Similarly, where reference is made to the presence of β-casein A¹ it encompasses phenotypes resulting from either a homozygous or heterozygous state with respect to the β-casein A¹ allele.

The term "processed dairy product(s)" is used herein to refer to dairy products derived from a source of bulk milk (i.e. from milk from more than one animal) and includes, but is not limited to:
(a) bulk milk used to make cheese whether or not the milk has been pasteurised or sterilised prior to cheese making,
(b) milk powder(s),
(c) milk fats,
(d) milk solids,
(e) casein(s), caseinate(s), and casein hydrolysates,
(f) pasteurised, sterilised, preserved milks including microfiltered milks, UHT milks,
(g) low fat milks,
(h) modified or enhanced milks,
(i) ice-cream or other frozen dairy based confections,
(j) fermented milk products such as yoghurt or quark,
(k) cheeses including full fat, partial de-fatted and fat-free processed cheeses,
(l) milk whey,
(m) food products enriched through the addition of milk products such as soups,
(n) milk from which allergenic molecules have been removed,
(o) confections such as chocolate,
(p) carbonated milk products, including those with added phosphate and/or citrate,
(q) infant formulations which may contain full, partially de-fatted or nonfat milk together with a number or additional supplements,
(r) liquid or powdered drink mixtures,
(s) butter, buttermilk, buttermilk powder.

### STATEMENT OF INVENTION

The invention provides a method for producing milk which is substantially free of the β-casein A¹ allele, or β-casein A¹ expressed therefrom.

Preferably the milk is derived from an animal which is substantially free of the β-casein A¹ allele.

The invention also provides a method for producing milk which contains the β-casein A² allele in preference to the β-casein A¹ allele.

The method of the invention provides a milk capable of minimising the onset of coronary heart disease characterised in that the milk is substantially free of β-casein A¹, or its proteolytic or heat produced products.

In a first aspect, the invention provides a method of producing milk suitable for use in the prevention of coronary heart disease from a herd of lactating bovines which milk is substantially free of β-casein A¹ the method including the steps of testing lactating bovines for the presence of the β-casein A¹ allele; selecting bovines which do not have the β-casein A¹ allele to form the herd of lactating bovines; and milking the selected bovines.

A second aspect provides the use of a milk product in the manufacture of an agent for the prevention of coronary heart disease, the milk product being obtained from milk of one or more lactating bovines, which milk is substantially free of β-casein A¹ and which milk is the product of a method according to the first aspect of the invention.

A third aspect of the invention provides a method of producing milk suitable for use in the prevention of coronary heart disease from a herd of lactating bovines, which milk is substantially free of β-casein A¹, the method including the steps of;
(i) screening of sperm from one or more bulls for the presence of the β-casein A¹ allele;
(ii) selecting sperm from the one or more bulls not having the β-casein A¹ allele; and
(iii) milking cows to give bulk milk, where the cows are the progeny of the one or more bulls having sperm selected as in step (ii) and cows which do not have the β-casein A¹ allele.

The invention provides a process for producing milk or milk products which does not contain β-casein A¹ by testing individual cows in a herd for the presence of the β-casein A¹ allele and utilising genetic engineering procedures to remove the β-casein A¹ allele or inhibit expression of β-casein A¹ therefrom.

The milk or milk product may be in the form of whole milk, whole-milk powder, skim milk, skim milk powder, milk whey, yoghurt, cheese, or any other dairy product, or processed dairy product.

The invention provides a method of reducing the onset of coronary heart disease in a human population which derives some of its food intake from milk or other diary products by reducing or substantially eliminating the presence of β-casein A¹ in the diet of that population.

### The discovery that is the basis of this Invention

It has been reported that certain groups of peoples are not subject to the diseases described above, notwithstanding the fact that they consume considerable quantities of milk proteins. These people include the Tibetans, rural Gambians, the Masai and Samburu people of Kenya. The latter peoples are also found not to suffer from obesity, even in old age. The only major difference between the milk consumed by the above people is that it is derived from Zebu, Bos Indicus, and Yak, Bos Mutus. Neither milk contains the casein allele described as β-casein A¹. In addition, people such as the Eskimo do not suffer from diseases such as CHD compared with their dairy product consuming Danish countrymen as is illustrated in Table 1:

**Table 1.**

| **Age-adjusted differences in morbidity from chronic diseases between Greenland Eskimos and Danes** | |
|---|---|
| | Eskimos/Danes |
| Acute myocardial infarction | 1/10 |
| Stroke | 2/1 |
| Psoriasis | 1/20 |
| Diabetes | rare |
| Bronchial asthma | 1/25 |
| Malignant disorders | 1/1 |
| Thyrotoxicosis | rare |
| Multiple sclerosis | 0 |
| Polyarthritis chronica | low |
| Acta Med Scand 208: 401-406, (1980) | |

These and other aspects of this invention, which should be considered in all its novel aspects, will become apparent from the following description, which is given by way of example only with reference to the preferred embodiments, and makes reference also to the following graphs:
**Figure 1** is a graph entitled "The effect of food component on Ischaemic Heart Disease during 1985 for males aged 30-69". This shows the death rate of all ages per 100,000 of population, for a range of countries, based on the consumption of β-casein.
**Figure 2** is a graph showing the effect of dairy protein consumption on Ischaemic Heart Disease for males aged 30-69 for the year 1985.
**Figure 3** is a graph showing the effect of saturated fat consumption on Ischaemic Heart Disease for males aged 30-69 for the year 1985.
**Figure 4** is a graph showing the effect of red meat consumption on Ischaemic Heart Disease for males aged 30-69 for the year 1985.

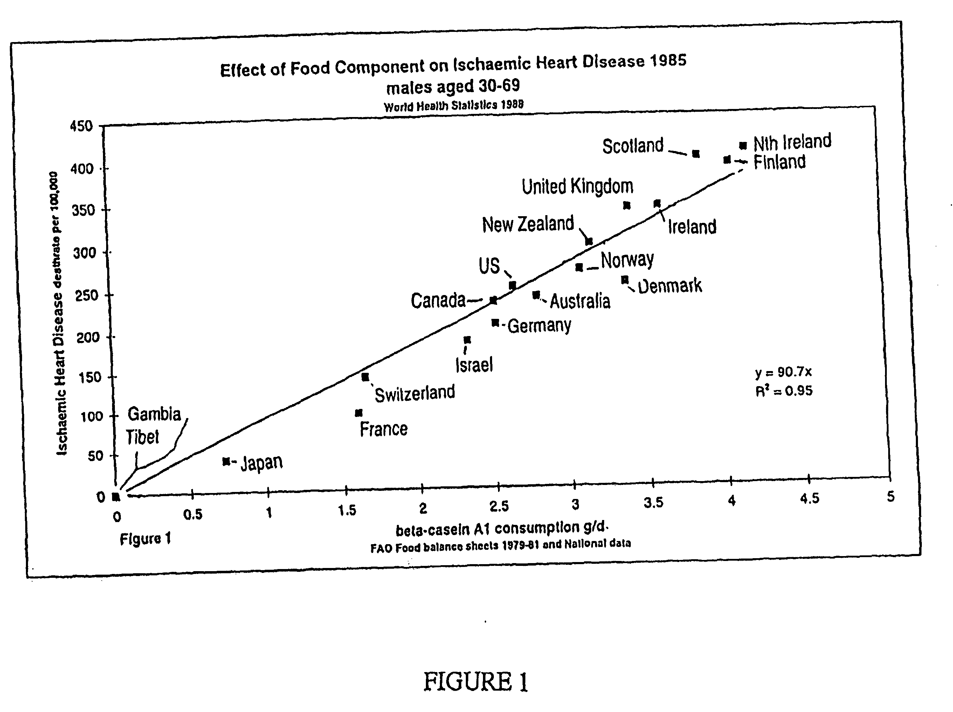

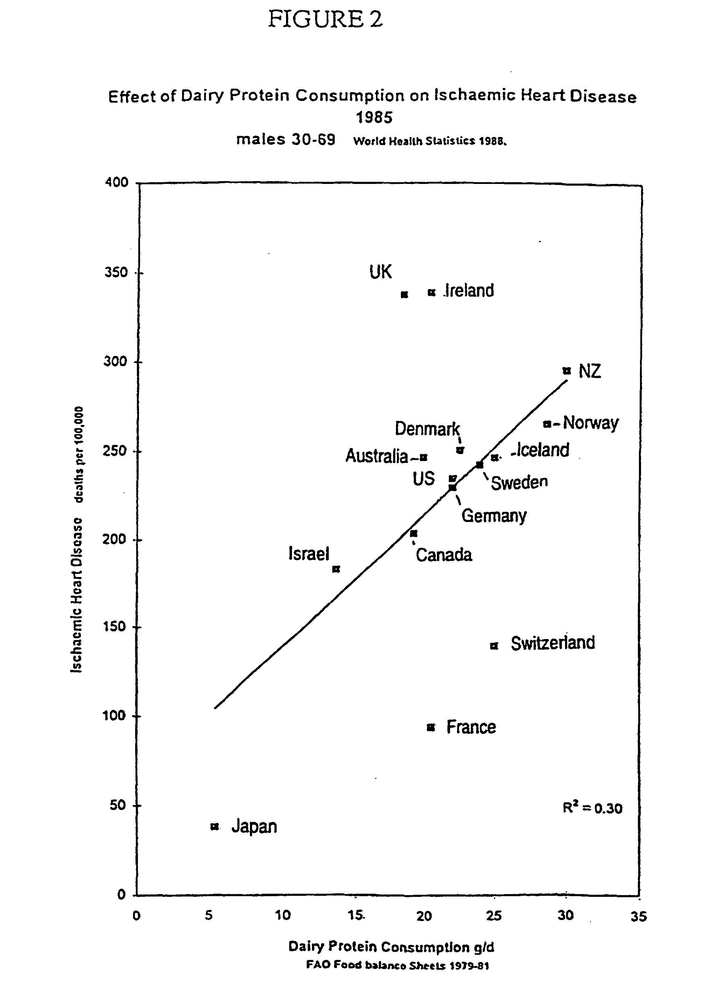

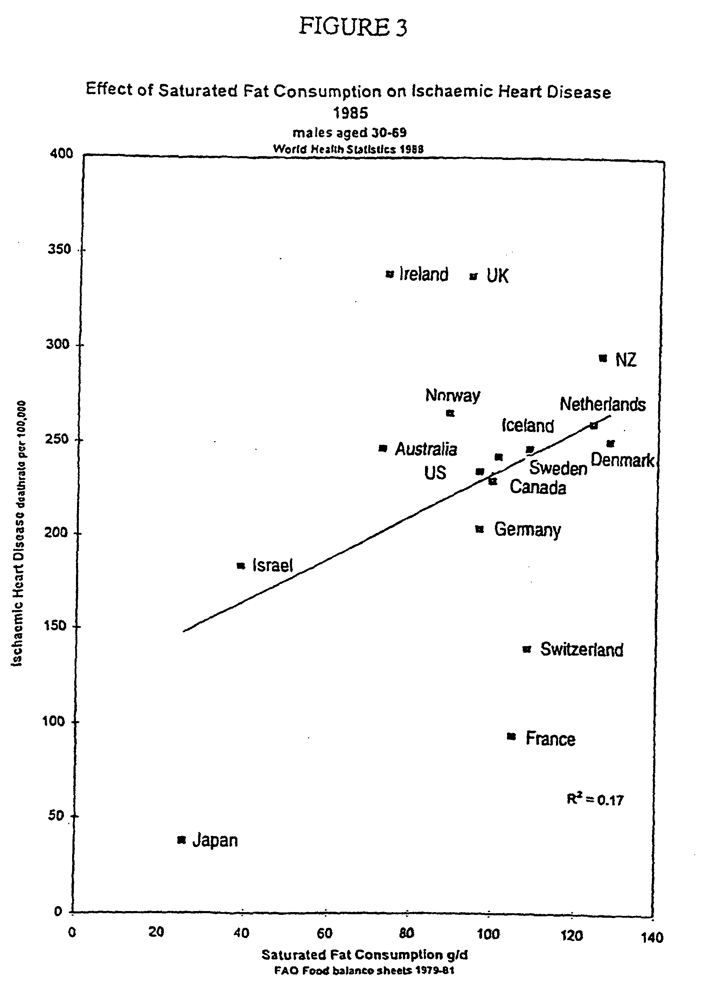

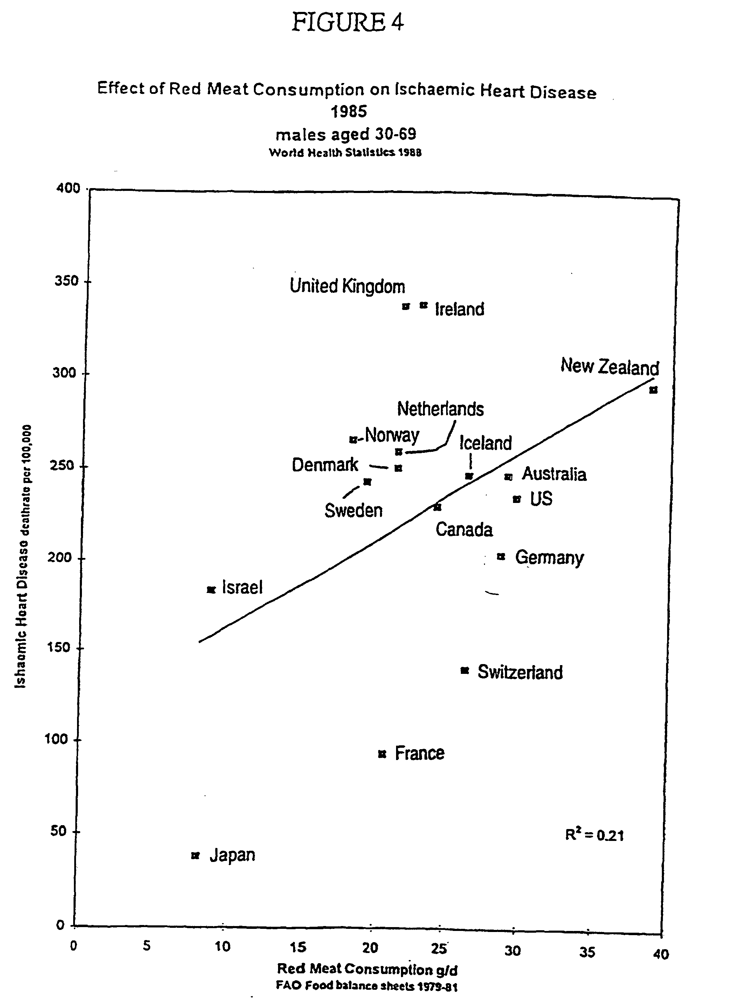

Figure 1 shows a very strong correlation between the consumption of the food component, identified as β-casein A¹ (discussed in more detail below), and the death rate. Whereas the overall dairy protein consumption (Figure 2) does not provide such a strong correlation nor does the effect of saturated fat consumption (Figure 3), nor the consumption of red meat (Figure 4) come anywhere close to the very strong correlation with the inventor has identified in relation to the consumption of β-casein A¹, both between countries and within countries. In the states of the form West Germany Ischaemic Heart Disease death rates are found to correlate directly with the consumption of β-casein A¹ (Table 1A). In this instance the composition of the state dairy herd have remained virtually constant from the 1950's through to the 1980's.

**Table 1A:**

| **CHD nutritional risk factors, Federal Republic of Germany based on Schleswig-Holstein** | | | | | | | |
|---|---|---|---|---|---|---|---|
| | Saturated Fat | Cholesterol | Alcohol | Carbohydrates | Energy | β-A¹ | Rd IHD est |
| Schleswig Holstein | 1.00 | 1.00 | 1.00 | 1.00 | 1.00 | 1.0 | 1.0 |
| Niedersachsen | 0.97 | 0.96 | 1.00 | 0.98 | 0.99 | 0.92 | 0.88 |
| Nordrhein Westfalen | 0.99 | 1.02 | 0.99 | 1.00 | 1.02 | 0.97 | 1.00 |
| Hessen | 0.95 | 0.96 | 0.98 | 0.98 | 0.98 | 0.75 | 0.74 |
| Rheinland-Pfalz | 0.95 | 0.99 | 1.00 | 1.02 | 1.0 | 0.87 | 0.78 |
| Saarland | 0.94 | 0.93 | 0.98 | 1.01 | 0.98 | 0.90 | 0.88 |
| Baden Wurttenburg | 0.93 | 1.02 | 1.02 | 1.05 | 1.03 | 0.50 | 0.72 |
| Bayem | 0.96 | 0.99 | 1.22 | 1.06 | 1.02 | 0.50 | 0.74 |

### DETAILED DESCRIPTION

Caseins constitute the majority of the milk proteins. Dairy cattle exhibit genetic polymorphism in their proteins. The heterogeneity of the caseins is further complicated by the fact that they are the products of co-dominant allele autosomal genes. Some indication of their number, and the major product fragments into which they are split by proteolytic action of a variety of enzymes, is illustrated by the β-caseins in Table 2.

**Table 2.**

| The β-casein family of proteins | | |
|---|---|---|
| Former nomen. | recommended nomen. | source of fragment |
| β-casein A¹ | β-CN A¹-5P | --- |
| β-casein A² | β-CN A²-5P | --- |
| β-casein A³ | β-CN A³-5P | --- |
| β-casein B | β-CN B-5P | --- |
| β-casein C | β-CN C-4P | --- |
| β-casein D | β-CN D-4P | --- |
| β-casein E | β-CN E-5P | --- |
| ³₁-casein A¹ | β-CN A¹-1P(f19-209) | β-CN A¹-5P |
| ³₁-casein A² | β-CN A²-1P(f29-209) | β-CN A²-5P |
| ³₁-casein A³ | β-CN A³-1P(f29-209) | β-CN A³-5P |
| ³₁-casein B | β-CN B-1P(f29-209) | β-CN B-5P |
| ³₂-casein A² | β-CN A² (f106-209) | β-CN A¹-5P or β-CN A²-5P |
| ³₂-casein A³ | β-CN A³(f106-209) | β-CN A³-5P |
| ³₂-casein B | β-CN B (f106-209) | β-CN B-5P |
| ³₃-casein A | β-CN A (f108-209) | β-CN A¹-5P, β-CN A²-5P or β-CN A³-5P |
| ³₃-casein B | β-CN B (f108-209) | β-CN B |
| In addition there are a number of proteose peptone components. | | |
| W N Eigel Nomenclature of Proteins of Cow's Milk: Fifth Revision | | |
| J. Dairy Science 67:1599-1631, (1984) | | |

Most animals are heterozygous. That is their protein composition contains a mixture of the various alleles inherited from the genes of their sire and dam. It appears that the original cow from which the current domesticated species developed contained only the β-casein A² allele. β-casein A¹ differs from A² in containing the replacement of amino acid proline 67 by a histidine. The corresponding A¹ allele is a relatively recent modification. However some animals are homozygous, that is their proteins are of one type only; in the case of β-caseins either A¹, A², A³, or B, C, D or E.

Bovine milk is an important source of proteins and other nutrients required by humans and the common domestic cattle species such as the Holstein have greater quantities of the A¹ allele than any other β-casein allele. Approximately 84 percent of the present American dairy herd it estimated to carry this allele.

In the graph shown in Figure 1 the cosumption of β-casein A¹ (and its derived proteolysis products) are plotted against the incidence of ischaemic heart disease based on FAO Food

Balance Sheets 1979-81 and WHO Trends in Mortality for Selected Causes of Death 1985-1989 and other reported CHD data.

In Table 3 the effect of heating milk to 63 °C for 20-30 minutes, known as Holder Pasteurisation, is set out together with the corresponding rate of CHD.

**Table 3.**

| **CHD rates following the Introduction of Holder Pasteurisation** | | | | | | | | | |
|---|---|---|---|---|---|---|---|---|---|
| Population group | Holder intro. year | Angina pectoris(AP1) mort. p mill. | | | | Cerebral embolism and thrombosis(CET) | | | |
| | | AP 1 | AP2 | AP3 | Δ% | CET 1 | CET 2 | CET 3 | Δ% |
| U.K | | | | | | | | | |
| Edinburgh | 1923 | 1925 | 67 | 92 | 37.3^{a} | 1924 | 174 | 236 | 35.6 |
| Glasgow | 1924 | 1924 | 56 | 91 | 62.5^{a} | 1924 | 77 | 101 | 31.2 |
| Dundee | 1924 | 1925 | 42 | 64 | 52.4^{a} | 1925 | 162 | 188 | 16.0 |
| Aberdeen | 1926 | 1926 | 91 | 135 | 48.4^{a} | 1927 | 121 | 227 | 87.6 |
| Lanarkshire | 1935 | 1937 | 188 | 375 | 99.5^{b} | 1938 | 153 | 193 | 26.1 |
| (excluding Glasgow) | 1947 | 1948 | 685 | 1185 | 73.0 | 1948 | 298 | 518 | 73.8 |
| | 1952 | 1954 | 1185 | 1523 | 28.5 | 1954 | 518 | 680 | 31.3 |
| County of Sutherland | 1954 | 1954 | 963 | 1710 | 77.9 | 1954 | 610 | 823 | 34.9 |
| County of Bute | 1956 | 1956 | 1610 | 2848 | 76.9 | 1956 | 955 | 1398 | 46.4 |

| London Admin. | | | | | | | | | |
|---|---|---|---|---|---|---|---|---|---|
| County | 1925 | 1925 | 31 | 112 | 261.3^{c} | 1926 | 90 | 120 | 33.3 |
| | Average increase | | | | 81.8 | | | | 41.6 |

| Norway | | | | | | | | | |
|---|---|---|---|---|---|---|---|---|---|
| Oslo | 1922 | 1922 | 3 | 43 | 1333.3^{d} | not available | | | |
| Columns AP1 and CET1 denote the year of commencement of the sudden nse in the appropriate mortality. | | | | | | | | | |
| Columns AP2 and CET2 denote the appropriate average mortality for the 4 years immediately preceding the year of introduction of pasteurisation. | | | | | | | | | |
| Columns AP3 and CET3 denote the appropriate average mortality for the 4 years immediately succeeding the introduction of pasteurisation. | | | | | | | | | |
| Δ% represents average increase. | | | | | | | | | |

| | | | | | | | | | |
|---|---|---|---|---|---|---|---|---|---|
| ^{a} Possibly low because deaths ascribed to "coronary thrombosis" were not included in International List No. 89 in Scotland until 1931. | | | | | | | | | |
| ^{b} Possibly enhanced as deaths ascribed to "coronary thrombosis" were now included in International List No. 94. | | | | | | | | | |
| ^{c} Possibly enhanced because (I) after 1927 all deaths ascribed to "coronary thrombosis" were included-unlike those in Scotland-in International list No. 89 and (ii) the large London creameries introduced Holder pasteurisation during this period. | | | | | | | | | |
| ^{d} Mortality ascribed to the following group of classifications: angina pectoris, infarctus cordis, sclerosis art. coron. cordis. | | | | | | | | | |

A proteolytic enzyme plasmin, which is naturally present in milk, and which is largely associated with the casein, is both increasingly active at higher temperatures and is quite heat stable. At 60 °C it has been demonstrated to have a relatively high rate of conversion of caseins, preferentially β-caseins to a range of proteolysis products. The increased mortality rate, demonstrated in Table 3, as a result of heating of the milk is presumed to be due to the formation of further proteolysis products, in addition to those naturally present, during the heating phase.

It is possible however that the specific fragment of β-casein A¹ that is entering or effecting the body's immune system which result from an enzyme contained within a psychotropic bacterium, or spore forming bacterium, present in the milk. Both the ratio of β-casein A¹/β-casein A² and the concentration of psychotropic bacteria vary seasonally in milk. This seasonal fluctuation is thought responsible for part of the seasonal fluctuation in the illnesses that we have noted above.

This work is further supported by the results of Bell Rc, Golemboski KA, Dietert RR, and Campbell TC, Nutrition and Cancer 22;(2),151-162,(1994) who found that when Fischer 344 rats were fed diets containing 6 percent and 22 percent casein after being injected with a liver cancer causing substance, aflatoxin, the percentage of animals developing liver cancer increased directly proportional to the increase in casein in the diet. They interpreted the results to suggest that a low protein diet might result in lower suppression of the natural killer cell cytotoxicity activity. With our knowledge we can re-interpret their data to suggest that based on our own observations on the effect of β-casein A¹ on immunosuppression in humans, its reduction in the rat's diet reduced cancer formation by a factor of four due to a dose specific effect on the rat's immune system.

The preferred forms of this invention comprises the elimination from milk of β-casein A¹ or its proteolysis products, or protein fragments formed in any other way, either 'in vitro' or 'in vivo' and which have immunosuppressant properties, by the use of immunoglobulins raised against β-casein A¹, the removal of β-casein A¹ and the inactivation of plasmin and other proteolytic enzymes. The preferred forms of the invention represents a significant advance over existing treatments for atherosclerosis, and other generally chronic immunosuppressant diseases in that it will prevent their occurrence in the new-born who, when they are genetically susceptible, will in other circumstances develop the diseases as they age. In addition it is believe it will assist in the restoration of organs and cells in those people where the damage to the bodies' organs is not permanent, by removing the source of chronic immune suppression.

By the term treatment, for the purpose of this invention it is intended that the symptoms of the disorder by ameliorated or completely eliminated or, where genetic typing indicates that an individual is of high risk of developing a disorder, of ensuring that it does not develop.

### Example 1 (Not an Embodiment of the Invention)

In its preferred form the treatment consists of inoculating a milk producing animal, that does not possess the β-casein A¹ allele, preferably one that is homozygous for the β-casein A² allele, preferably one that produces commercially feasible quantities of milk, such as a cow, sheep, goat, or zebu with β-casein A¹, or its proteolysis products, or fragments thereof, produced in any other manner, so that antigens to the foreign β-casein A¹ protein are produced. These antigems may be produced either alone, or as part of a wider inoculation programme, to produce a milk with an enhanced antigen concentration as has been described in the Art. This antigen enhanced milk is then added to 'normal' milk, or milk, or milk products, whose β-casein A¹ content has been reduced, using techniques known to those skilled in the Art, to counteract the presence of the immune suppressing β-casein A¹ derived material. Alternatively the above antigen(s) may be recovered by one of the processes known to the Art and used as a food supplement in its own right either alone or as a food additive.

Because the immunoglobulins formed as a result of the inoculation programme are somewhat heat sensitive then care has to exercised with the pasteurisation and handling of the final product if a powder is required as is described in the existing Art.

Alternatively, immunoglobulins and other antigens, are recovered from non β-casein A¹ containing milk by ultrafiltration, ion exchange chromatography either singly, or in combination, or by use of a suitable immunoadsorbent column, comprising an insoluble carrier material to which is bound a low-affinity monoclonal antibody specific to one or more milk immunoglobulins but not specific to any other common constituent of milk. Such milk may having been derived from an animal that has been inoculated with a vaccine derived from a bacteria such as E. coli, for example, or which has been inoculated with 'bacterial antigens", as described in the Art. Alternatively, enhanced quantities of antigens are produced as a result of the inoculation, or inoculation programme of β-casein A¹-free animals, to provide a milk product with all the claims as described in the prior Art. This invention has the advantage over the existing Art that immunosuppressant proteins resulting from the presence of, or, derived from the β-casein A¹ allele are eliminated from the final milk, or milk-derived products.

Another alternative includes the use of a plasmin inhibitor, such as a protein like aprotonin, or other such inhibitors, known to the Art, which are added, either singly or in mixtures, to the milk, as part of the above invention, to suppress the formation of additional β-casein A¹ proteolysis products that would otherwise be formed during processing, and storage, prior to sale.

### Example 2

A milk or other dairy product according to the invention can be produced by testing individual cows in a dairy herd for the presence of the β-casein A¹ allele, or for the presence of β-casein A¹ in milk, and then selectively culling those cows returning a positive result, until the bulk milk produced by the herd is substantially free of β-casein A¹. Alternatively, homozygous cattle containing the β-casein A² allele can be selectively bred so that the β-casein A¹ allele is eliminated from the herd.

An alternative approach to remove β-casein A¹ from bulk milk would involve separating cattle from existing herds which contain the β-casein A¹ allele, allowing the remainder of the herd (which are free of the β-casein A¹ allele) to be used for the production of bulk milk or other dairy products, and those cattle containing the A¹ allele to be used for the production of products for purposes other than human consumption. Such a segregation process within a herd may be facilitated by the use of ear tags or the like to mark individual animals.

### Industrial Application

The invention provides a useful food product capable of increasing the health of an individual, or the health of a population. In one aspect the invention provides a method of enhancing the immune response of an individual or a population who or which derives some of his/her/its food intake from milk or other diary products, by reducing or substantially eliminating the presence of β-casein A¹ in the diet of that individual or that population.

In a particularly preferred form, the invention applies a method of reducing the onset of coronary heart disease in a human population which derives some of its food intake from milk or other diary products by reducing or substantially eliminating the presence of β-casein A¹ within the diet of that population.

### ADVANTAGES

By reducing or substantially eliminating the presence of β-casein A¹ in the diet of humans, it is believed that the immune response of an individual or a population may be enhanced, or immunosuppression reduced, increasing the general well-being of the individual or the population. It is believed that some individuals may be particularly susceptible to the presence of β-casein A¹, and it may be possible to develop a test for such susceptible individuals, and to recommend that they reduce or eliminate their consumption of milk or other diary products containing β-casein A¹.

### VARIATIONS

Recognising that dairy products free of β-casein A¹ are desirable it is preferable to ensure that the animal from which the product is derived has been tested for the presence of the β-casein A¹ allele or β-casein A¹ expressed therefrom and subsequent selective breeding programmes (selecting for β-casein A¹ negative animals) carried out to eliminate the presence of the β-casein A¹ from the herd.

An alternative approach to remove the β-casein A¹ allele from a herd may be carried out. Such an approach may include the screening of sperm to be used for the purpose of artificial insemination for the presence or absence of the β-casein A¹ allele and selecting against those sperm which contain this allele.

In addition to the methods of removing β-casein A¹ (or the β-casein A¹ allele), from milk and "processed dairy products" that have been disclosed herein it would be within the scope of this invention to use a number of alternative methods. Such alternative methods may involve the removal of β-casein A¹ from milk products via ultrafiltration techniques or by utilising a non-toxic chemical or enzymatic process to remove or inactivate β-casein A¹.

## Claims

1. A method of producing milk suitable for use in the prevention of coronary heart disease from a herd of lactating bovines which milk is substantially free of β-casein A¹, the method including the steps of:
(i) testing lactating bovines for the presence of the β-casein A¹ allele;
(ii) selecting bovines which do not have the β-casein A¹ allele to form the herd of lactating bovines; and
(iii) milking the selected bovines.

2. A method of producing milk as claimed in claim 1 in which the lactating bovines tested in step (i) are, or includes, *Bos taurus* bovines.

3. A method as claimed in claim 2 wherein the lactating bovines are *Bos taurus* bovines.

4. The use of a milk product in the manufacture of an agent for the prevention of coronary heart disease, the milk product being obtained from the milk of one or more lactating bovines, which milk is substantially free of β-casein A¹, and which milk is the product of a method including the steps of:
(i) testing the one or more lactating bovines for the presence of the β-casein A¹ allele;
(ii) selecting bovines which do not have the β-casein A¹ allele; and
(iii) milking the selected bovines.

5. A method of producing milk suitable for use in the prevention of coronary heart disease from a herd of lactating bovines which milk is substantially free of β-casein A¹, the method including the steps of:
(i) screening of sperm from one or more bulls for the presence of the β-casein A¹ allele;
(ii) selecting sperm from the one or more bulls not having the β-casein A¹ allele; and
(iii) milking cows to give bulk milk, where the cows are the progeny of the one or more bulls having sperm selected as in step (ii) and cows which do not have the β-casein A¹ allele.

## Patentansprüche

1. Verfahren zur Herstellung von Milch, geeignet zur Verwendung bei der Prävention von koronarer Herzkrankheit, von einer Herde laktierender Rinder, wobei die Milch im wesentlichen frei von β-Kasein A¹ ist, wobei das Verfahren die Schritte umfaßt:
(i) Testen von laktierenden Rindern auf das Vorhandensein von β-Kasein A¹ Allelen;
(ii) Auswählen von Rindern, die keine β-Kasein A¹ Allele aufweisen, um die Herde von laktierenden Rindern zu bilden; und
(iii) Melken der ausgewählten Rinder.

2. Verfahren zur Herstellung von Milch gemäß Anspruch 1, bei dem die in Schritt (i) getesteten Rinder *Bos taurus* Rinder sind oder umfassen.

3. Verfahren gemäß Anspruch 2, bei dem die laktierenden Rinder *Bos taurus* Rinder sind.

4. Verwendung eines Milchprodukts bei der Herstellung eines Mittels zur Prävention von koronarer Herzkrankheit, wobei das Milchprodukt erhalten wird von der Milch von einem oder mehreren laktierenden Rindern, wobei die Milch im wesentlichen frei von β-Kasein A¹ ist und die Milch das Produkt eines Verfahrens ist, umfassend die Schritte:
(i) Testen von ein oder mehreren laktierenden Rindern auf das Vorhandensein des β-Kasein A¹ Alleles;
(ii) Auswählen von Rindern, die nicht das β-Kasein A¹ Allel aufweisen; und
(iii) Melken der ausgewählten Rinder.

5. Verfahren zur Herstellung von Milch, geeignet zur Verwendung bei der Prävention von koronarer Herzkrankheit, aus einer Herde von laktierenden Rindern, wobei die Milch im wesentlichen frei von β-Kasein A¹ ist, und das Verfahren die Schritte umfasst:
(i) Screening des Spermas von einem oder mehreren Bullen auf das Vorhandensein des β-Kasein A¹ Alleles;
(ii) Auswählen von Sperma von einem oder mehreren Bullen, die nicht das β-Kasein A¹ Allel aufweisen; und
(iii) Melken von Kühen, um Rohmilch zu ergeben, wobei die Kühe die Nachkommen der ein oder mehreren Bullen sind, die Sperma aufweisen, ausgewählt wie in Schritt (ii) und wobei die Kühe nicht das β-Kasein A¹ Allel aufweisen.

## Revendications

1. Un procédé de production de lait approprié à l'utilisation dans la prévention de maladie coronarienne à partir d'un troupeau de bovins allaitant pour lequel le lait est essentiellement libre de β-caséine A¹, le procédé incluant les étapes de :
(i) essai de bovins allaitant pour la présence de l'allèle β-caséine A¹ ;
(ii) sélection des bovins qui n'ont pas l'allèle β-caséine A¹ pour former le troupeau de bovins allaitant; et
(iii) traite des bovins sélectionnés.

2. Un procédé de production de lait selon la revendication 1 dans lequel les bovins allaitant testés dans l'étape (i) sont ou incluent les bovins *Bos taurus*.

3. Un procédé selon la revendication 2 dans lequel les bovins allaitant sont des bovins *Bos taurus*.

4. L'utilisation d'un produit laitier dans la fabrication d'un agent pour la prévention de maladie coronarienne, le produit laitier étant obtenu à partir du lait d'un ou plusieurs bovins allaitant, dont le lait est essentiellement libre de β-caséine A¹, et dont le lait est un produit d'un procédé incluant les étapes de :
(i) essai d'un ou plusieurs bovins allaitant pour la présence de l'allèle de β-caséine A¹;
(ii) sélection des bovins qui n'ont pas l'allèle de β-caséine A¹;et
(iii) traite des bovins sélectionnés.

5. Un procédé de production de lait approprié pour l'utilisation dans la prévention de maladie coronarienne à partir d'un troupeau de bovins allaitant dont le lait est essentiellement libre de β-caséine A¹, le procédé incluant les étapes de :
(i) dépistage de sperme d'un ou plusieurs taureaux pour la présence de l'allèle de β-caséine A¹;
(ii) sélection du sperme d'un ou plusieurs taureaux n'ayant pas l'allèle de β-caséine A¹; et
(iii) traite des vaches pour donner du lait en bloc, où les vaches sont la progéniture d'un ou plusieurs taureaux ayant le sperme sélectionné comme dans l'étape (ii) et de vaches n'ayant pas l'allèle de β-caséine A¹.
